# EUROPÄISCHE PATENTSCHRIFT

(11) **EP 1 761 292 B1**
(45) Veröffentlichungstag und Bekanntmachung des Hinweises auf die Patenterteilung: **21.09.2011**
(21) Anmeldenummer: 05761193.1
(22) Anmeldetag: 07.06.2005
(51) Int. Cl.: A61M 16/04

(54) **Vorrichtung zur Verbindung eines Beatmungsgerätes mit dem Patienten**
Device for connecting a respirator to a patient
Dispositif pour relier un appareil respiratoire au patient

(30) Priorität: 07.06.2004 DE 102004027734
(43) Veröffentlichungstag der Anmeldung: 14.03.2007
(73) Patentinhaber: Filtertek B.V., County of Limerick (IE)
(72) Erfinder: MIJERS, Jan Willem Marinus, NL-2014 AL Haarlem (NL)
(74) Vertreter: Beck, Alexander
(86) Internationale Anmeldenummer: PCT/EP2005/006114
(87) Internationale Veröffentlichungsnummer: WO 2005/120618

(56) Entgegenhaltungen:
- DE-A1- 3 327 342
- DE-A1- 3 435 900
- US-A- 3 794 043
- US-A- 4 825 862

## Beschreibung

Die Erfindung betrifft eine Vorrichtung zur Verbindung eines Beatmungsgeräts mit dem Patienten mit einem in die Trachea des Patienten einführbaren, mit dem Atemgas beaufschlagbaren Beatmungstubus, wie Trachealtubus bzw. Tracheostomietubus, der nahe dem distalen Ende mit einem aufblasbaren Cuff versehen ist, welcher eine Verbindungsleitung zum proximalen Ende aufweist, die mit einem elastischen Druckluftspeicher verbunden ist, wobei ein mindestens drei Wege aufweisendes Ventil zwischen dem proximalen Ende der Verbindungsleitung und dem Druckluftspeicher angeordnet ist, welches einen Eingang mit Anschluss für eine Injektionsspritze zum Füllen des Cuffs und des Druckluftspeichers mit Druckluft aufweist und das Ventil an dem Eingang als Rückschlagventil und zur Aufrechterhaltung eines konstanten Drucks in dem Cuff mittels Ausgleich zwischen Cuff und dem Druckluftspeicher als Steuerventil wirksam ist.

Eine derartige Vorrichtung ist beispielsweise in dem US-Patent 3 794 043 beschrieben und unter der Bezeichnung "Lanz-System" auf dem Markt erhältlich. Eine derartige Vorrichtung wird zum Beatmen von Patienten verwendet, wobei, nachdem der Beatmungstubus in die Trachea des Patienten eingeführt wurde, üblicherweise eine Injektionsspritze verwendet wird, um den am distalen Ende des Beatmungstubus angeordneten Cuff aufzublasen, wobei in dem Cuff ein Druck zwischen 22 und 25 mm Hg (30 bis 34 cm Wassersäule) erreicht werden sollen. Gleichzeitig wird hierbei auch der Druckluftspeicher gefüllt. Nach Entfernen der Injektionsspritze wird ein konstanter Druck in dem Cuff durch den Druckausgleich zwischen Cuff und Druckluftspeicher aufrechterhalten, indem dieser Druckausgleich durch das Ventil gesteuert wird. Die Anordnung ist hierbei derart getroffen, dass die Luft von dem Druckluftspeicher in den Cuff vergleichsweise leicht zurückströmen kann, während die Luft in umgekehrter Richtung, d.h. von dem Cuff in den Druckluftspeicher, mit einer reduzierten Strömungsgeschwindigkeit strömen soll, wodurch verhindert werden soll, dass Leckverluste zwischen Cuff und Trachea auftreten. Die bekannte Vorrichtung dieser Art arbeitet zwar zufriedenstellend, weist jedoch den erheblichen Nachteil auf, dass sie aufgrund der Komplexität und der zum Teil verwendeten empfindlichen Werkstoffe ausgesprochen aufwändig herzustellen ist. So besteht beispielsweise bei der bekannten Vorrichtung das Ventil aus zwei ineinander geschobenen Gehäusen, von denen das Innere einen pilzförmigen Körper aus Naturkautschuk als Ventil aufnimmt, dessen Unterende die Dichtfläche des Rückschlagventils bildet, welches den Eingang steuert. Zur Betätigung des Rückschlagventils ist ein gesonderter Stößel aus Kunststoff vorgesehen, welcher beim Befüllen durch die Injektionsspritze verschoben werden muss, um das Ventil zu öffnen. Der Druckluftspeicher ist als Blase aus Naturkautschuk ausgebildet, welche noch durch eine äussere Hülle aus Weichplastik gegen Beschädigungen geschützt ist. Diese Kautschukblase wird an dem Ventilkörper zwischen den beiden ineinander geschobenen Gehäusen eingeklemmt und befestigt. Der innerhalb der Kautschukblase angeordnete Kopf des pilzförmigen Ventilkörpers bildet in der Kombination seines auf der Ventiloberseite aufliegenden Randes und Entlastungsöffnungen in dem Kopf den Steuerventilteil. Es ist offensichtlich, dass sowohl die Verwendung von Naturkautschuk als auch die große Anzahl von Einzelteilen nachteilig ist.

Der Erfindung liegt daher die Aufgabe zu Grunde, ausgehend von einer Vorrichtung der eingangs beschriebenen Art, diese Nachteile zu vermeiden und insbesondere eine hohe Funktionssicherheit bei geringen Herstellungskosten zu gewährleisten.

Diese Aufgabe wird erfindungsgemäß im Wesentlichen dadurch gelöst, dass das Ventil als Dreiwege-Rückschlag- und als Differenzdruckventil ausgebildet ist, wobei das Ventil mindestens zwei Ausgänge mit je einer zugeordneten Differenzkraftkammer aufweist und der Eingang mit einer dritten Differenzkraftkammer versehen ist, dass die Differenzkraftkammer durch eine elastische Membranscheibe dichtend voneinander getrennt sind, und dass der erste Ausgang an die Verbindungsleitung und der zweite Ausgang an den Druckluftspeicher angeschlossen ist.

Es ist offensichtlich, dass bereits durch die Ausgestaltung des Ventils als einfaches eine Membran enthaltendes Ventil eine sehr viel vereinfachte Konstruktion geschaffen wird. Durch die Verwendung von durch die Membranscheibe getrennten Differenzkraftkammern ist die gewünschte Funktion ebenfalls leicht erreichbar, indem durch entsprechende Wahl der Flächenanteile der Membran, die der jeweiligen Differenzkraftkammer zugeordnet sind, ein schnelles und leichtes Rückströmen der Luft aus dem Druckluftspeicher und ein langsameres Strömen der Luft aus dem Cuff in den Druckluftspeicher erreicht werden kann.

Bei einer besonders bevorzugten Ausführungsform nach der Erfindung ist die erste Differenzkraftkammer des ersten Ausgangs mit einem Entlastungsventil verbunden, wobei insbesondere bevorzugt ist, das Entlastungsventil zum Entspannen des Cuffs durch an dessen Ausgang erzeugten Unterdruck betätigbar zu machen. Hierdurch werden besondere Stößel oder mechanische Einrichtungen vermieden, nachdem der Cuff einfach durch Erzeugung eines Unterdrucks entlastet werden kann.

Im Einzelnen kann die Erfindung dadurch weitergebildet werden, dass das Differenzdruckventil aus zwei dichtend miteinander verbindbaren ventilgehäusehälften gebildet ist, wobei die erste Ventilgehäusehälfte den Eingang und die zweite Ventilgehäusehälfte die beiden Ausgänge und das Entlastungsventil enthält.

Die Membranscheibe ist hierbei bevorzugt zwischen der ersten und der zweiten Ventilgehäusehälfte eingespannt. Hierdurch wird die Herstellung des Ventils stark vereinfacht.

Eine besonders bevorzugte Ausführungsform nach der Erfindung kann dadurch geschaffen werden, dass das erste Ventilgehäuse innerhalb der ersten Differenzkraftkammer einen Vorsprung aufweist, welcher auf seinem Oberende einen ringförmigen Steg trägt, der die Membranscheibe in Richtung des ersten Ausgangs vorspannt und eine in der Membranscheibe vorgesehene Öffnung umgibt, wobei radial ausserhalb des Vorsprungs Verbindungskanäle zwischen dem Einlass und der ersten Differenzkraftkammer vorgesehen sind, und dass die erste Differenzkraftkammer durch einen zweiten ringförmigen Steg mit größerem Durchmesser als der erste Ringsteg am Umfang begrenzt ist, welcher die Membranscheibe in Richtung des ersten Ausgangs vorspannt.

Im Einzelnen ist es vorteilhaft, dass der erste Ausgang parallel oder koaxial zum Eingang angeordnet ist.

Eine vorteilhafte Weiterbildung nach der Erfindung kann dadurch geschaffen werden, dass radial ausserhalb des zweiten Ringstegs mit größerem Durchmesser ein Ringraum vorgesehen ist, welcher über Öffnungen in dem Membranscheibe mit der dem zweiten Ausgang zugeordneten zweiten Differenzkraftkammer verbunden ist.

Eine besonders vorteilhafte Ausbildungsform nach der Erfindung kann dadurch geschaffen werden, dass in der dem ersten Ausgang zugeordneten ersten Differenzkraftkammer ein den Auslass umgebender dritter ringförmiger Steg vorgesehen ist, welcher die Membranscheibe in Richtung des Eingangs vorspannt und dessen Durchmesser größer als der Durchmesser des ersten Ringstegs und kleiner als der Durchmesser des zweiten Ringstegs ist. Durch diese Beziehung der durch die Durchmesser der drei verschiedenen Ringstege festgelegten wirksamen Flächen der Membran wird auf einfachstem Wege das gewünschte Ansprechverhalten des Ventils bestimmt.

Im Einzelnen ist es von Vorteil, dass das Entlastungsventil in einem, einen Anschluss aufweisenden, mit der zweiten Ventilgehäusehälfte verbundenen Gehäuse angeordnet ist.

Hierbei ist es von Vorteil, dass der Anschluss des Gehäuses und der Anschluss des Eingangs zum Einführen des Vorderendes einer Injektionsspritze ausgebildet sind. Durch diese Merkmale wird erreicht, dass auch das Entlasten des Cuffs mittels einer einfachen Injektionsspritze erfolgen kann, indem diese den zur Betätigung des Entlastungsventils notwendigen Unterdruck erzeugt.

Eine besonders bevorzugte Ausführungsform kann dadurch geschaffen werden, dass der Druckluftspeicher als mit dem zweiten Ausgang verbundener Gummibalg ausgebildet ist, welcher bevorzugt in einem Gehäuse aufgenommen ist. Hierdurch wird eine besonders vorteilhafte Ausgestaltung des Druckluftspeichers geschaffen.

Zur weiteren Vereinfachung der Herstellung ist es ferner bevorzugt, dass zumindest der Boden des Gehäuses einstückig mit der zweiten Ventilgehäusehälfte ausgebildet und über ein einen Verbindungskanal zum zweiten Ausgang umgebendes Kupplungsstück mit dem Gummibalg verbunden ist.

Ferner ist es bevorzugt, dass die Auslassöffnung der zweiten Differenzkraftkammer abgewinkelt zum Verbindungskanal verläuft, da hierdurch eine besonders kompakte Bauweise ermöglicht wird.

Ferner kann die Erfindung dadurch weitergebildet werden, dass die Membranscheibe am Umfang einen Ringwulst aufweist, welcher in einander gegenüberliegenden ringförmigen Ausnehmungen der Ventilgehäusehälften aufgenommen ist. Hierdurch wird eine exakte und leichte Endmontage der erfindungsgemäßen Vorrichtung erreicht, da diese Ausgestaltung eine exakte Positionierung und Halterung der Membranscheibe ermöglicht.

Im Einzelnen.ist es von Vorteil, dass das Entlastungsventil als Pilzventil ausgebildet ist, dessen elastische Membran ein oder mehrere Verbindungskanäle zur ersten Differenzkraftkammer abdeckt. Diese Ausgestaltung stellt eine besonders einfache Konstruktion des Entlastungsventils dar.

Im. Folgenden wird die Erfindung an Hand einer in den Zeichnungen beispielhaft veranschaulichten Ausführungsform näher erläutert. Es zeigt:
FIGUR 1 eine schematische teilweise geschnittene Gesamtansicht der Vorrichtung nach der Erfindung;
FIGUR 2 eine Aussenansicht des Ventils mit dem Druckluftspeicher in Richtung des Pfeiles II in Figur 1;
FIGUR 3 eine Schnittansicht längs der Linie III-III in Figur 2 und
FIGUR 4 eine vergrößerte Schnittansicht einer Einzelheit von Figur 3.

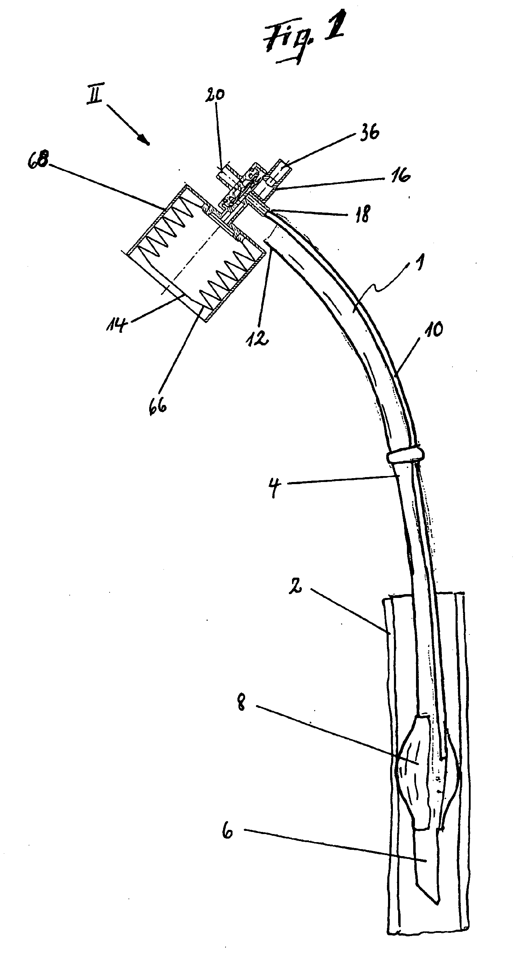

In Figur 1 der Zeichnungen ist in einer Ausführungsform der allgemein mit 1 bezeichneten Vorrichtung zur Verbindung eines nicht dargestellten Beatmungsgeräts mit einem Patienten dargestellt, welcher einen in die Trachea 2 des Patienten einführbaren und mit Atemgas beaufschlagbaren flexiblen Beatmungstubus 4 aufweist, bei welchem es sich um einen Trachealtubus bzw. Tracheostomietubus handeln kann. Durch den Beatmungstubus wird in üblichen periodischen Abständen Luft in die Lungen eingepresst. Nahe seinem, im Inneren der Trachea 2 liegenden oder distalen Ende 6 ist der Beatmungstubus 4 mit einem aufblasbaren Cuff 8 versehen, welcher das distale Ende 6 umgibt und um dieses eine Luftkammer bildet. Im Ausgangszustand ist der Cuff 8 normalerweise flach an dem Beatmungstubus 4 mehr oder weniger anliegend, so dass er und der Beatmungstubus 4 in die Trachea 2 eingeführt werden kann. Der Chirurg wählt hierzu einen Durchmesser des Beatmungstubus 4 auf der Basis seiner Einschätzung des Durchmessers der Trachea. Um den Cuff 8 zur Ausbildung einer Dichtung mit der Wandung der Trachea 2 aufzublasen, ist eine sehr viel dünnere Verbindungsleitung 10 mit dem Cuff verbunden und die zum proximalen Ende 12 führt und ihrerseits mit einem elastischen Druckluftspeicher 14 verbunden ist.

Ein mindestens drei Wege aufweisendes Ventil 16 ist zwischen dem proximalen Ende 18 der Verbindungsleitung 10 und dem Druckluftspeicher 14 angeordnet.

Durch das Ventil kann Druckluft durch irgend eine zweckdienliche Vorrichtung zugeführt werden, wobei eine übliche Injektionsspritze bevorzugt ist. Das Ventil 16 weist hierzu einen Eingang 20 auf, der mit einem entsprechend gestalteten Anschluss 64 für die Injektionsspritze versehen ist, durch welche sich der Cuff 8 und der Druckluftspeicher 14 durch entsprechende Betätigung der Spritze gefüllt werden kann. Am Eingang 20 ist das Ventil 16 als Rückschlagventil wirksam, welches sich schließt, wenn die Injektionsspritze entfernt wird, um zu verhindern, dass Luft aus dem Cuff wieder ausströmen kann. Gleichzeitig verbindet das Ventil 16 den Cuff 8 mit dem Druckluftspeicher 14 und wird hier als Steuerventil wirksam, welches einen konstanten Druck des Cuffs 8 gewährleistet, indem durch Betätigung des Steuerventils in der einen oder anderen Richtung jeweils Luftmengen zwischen dem Cuff 8 und dem Druckluftspeicher 14 verschoben werden.

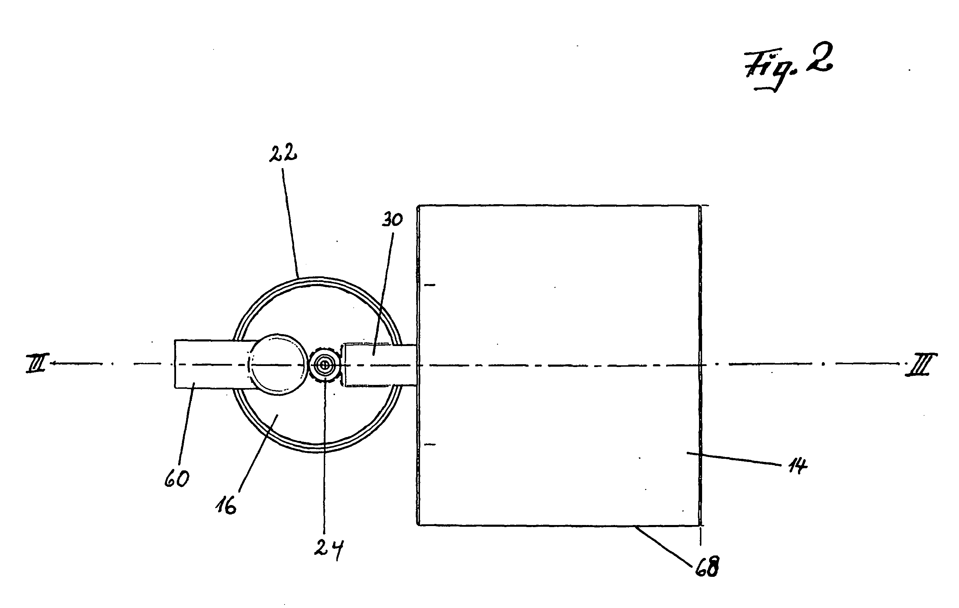

Erfindungsgemäß ist das Ventil 16 als Dreiwege-Rückschlag- und als Differenzdruckventil 22 ausgebildet und zur näheren Erläuterung wird im Folgenden auf die Figuren 2 bis 4 Bezug genommen.

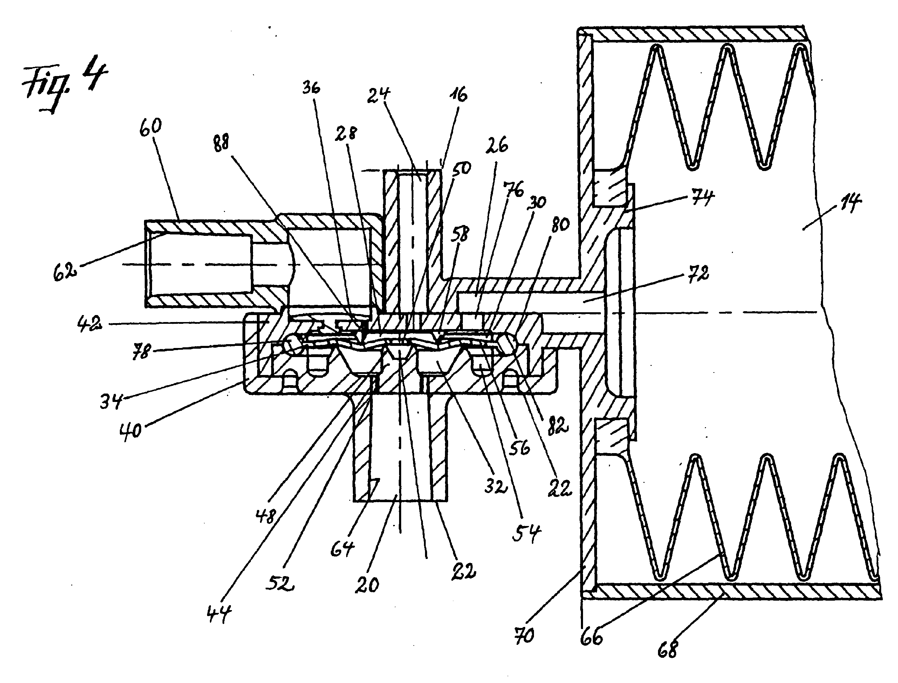

Wie insbesondere aus Figur 4 ersichtlich, weist das Ventil 22 zwei Ausgänge 24, 26 auf, denen je eine Differenzkraftkammer 28 bzw. 30 zugeordnet ist. Dem Eingang 20 ist eine dritte Differenzkraftkammer 32 zugeordnet.

Die Differenzkraftkammern 28, 30 und 32 sind durch eine Membranscheibe 6 voneinander abgedichtet getrennt.

Der erste Ausgang 24 ist an die Verbindungsleitung 10 und der zweite Ausgang 26 an den Druckluftspeicher 14 angeschlossen.

Zusätzlich ist die erste Differenzkraftkammer 28 des ersten Ausgangs 24 mit einem, allgemein mit 36 bezeichneten Entlastungsventil verbunden, welches einen Ausgang 38 aufweist, über den das Entlastungsventil 36 zum Entspannen des Cuffs 8 durch Unterdruck betätigbar ist, was weiter unten noch näher erläutert wird.

Das Gehäuse des Differenzdruckventils 22 besteht aus zwei dichtend miteinander verbundenen Ventilgehäusehälften 40 und 42. Die erste Ventilgehäusehälfte 40 enthält den Eingang 20 und die zweite Ventilgehäusehälfte 42 die beiden Ausgänge 24 und 26 sowie das Entlastungsventil 36. Zwischen der ersten und der zweiten Ventilgehäusehälfte 40 und 42 ist die Membranscheibe 34 eingespannt.

Innerhalb der ersten Differenzkraftkammer 28 weist die erste Ventilgehäusehälfte 40 einen zapfenartigen Vorsprung 44 auf, auf dessen Oberende 46 ein erster ringförmiger Steg 48 ausgebildet ist. Mittels des Vorsprungs 44 und des ringförmigen Stegs 48 wird die Membranscheibe 34 in Richtung des ersten Ausgangs 24 vorgespannt, wobei der Ringsteg 48 eine in der Membranscheibe 34 vorgesehene Öffnung 50 umgibt.

Radial ausserhalb des Vorsprungs 44 sind Verbindungskanäle 52 in der einstückig durch Spritzgießen aus Kunststoff hergestellten ersten Ventilgehäusehälfte 40 ausgebildet, welche eine Verbindung zwischen dem Einlass 20 und der ersten Differenzkraftkammer 28 herstellen. Die erste Differenzkraftkammer 28 wird weiterhin durch einen zweiten ringförmigen Steg 53, welcher ebenfalls einstückig mit der ersten Ventilgehäusehälfte 40 ausgebildet ist und welcher einen größeren Durchmesser als der erste Ringsteg 48 aufweist, am Umfang begrenzt. Der zweite Ringsteg 53 spannt ebenfalls die Membranscheibe 34 in Richtung des ersten Ausgangs vor.

Die dritte Differenzkraftkammer 32 wird somit durch den Ringraum zwischen dem ersten Ringsteg 48 und dem zweiten Ringsteg 53 und die auf diesen aufliegende Teile der Membranscheibe 34 definiert und bildet die Einlasskammer beim Füllen des Cuffs 8 und des Druckluftspeichers 14, wobei der die dritte Differenzkraftkammer 32 schließende Streifen der Membranscheibe 34 die Funktion des Rückschlagventils übernimmt.

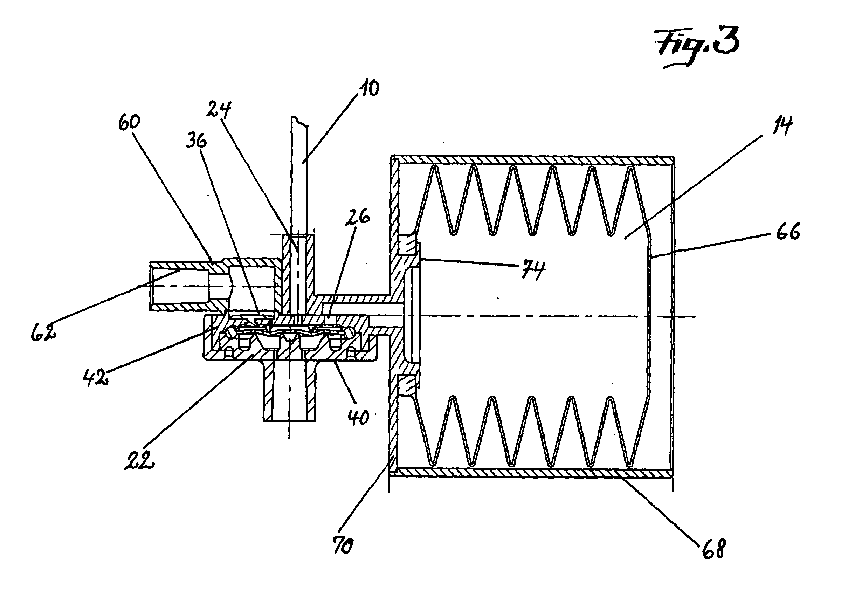

Wie insbesondere aus Figuren 3 und 4 ersichtlich, ist der erste Ausgang 24 parallel oder koaxial zum Eingang 20 angeordnet, wenn die beiden Ventilgehäusehälften 40 und 42 miteinander verbunden sind.

In der ersten Ventilgehäusehälfte 40 ist ferner ausserhalb des zweiten Ringstegs 53 mit größerem Durchmesser ein Ringraum 56 ausgebildet, welcher über entsprechende, auf diesem Radius angeordnete Öffnungen 56 in der Membranscheibe 34 mit der zweiten Differenzkraftkammer 30 in Verbindung steht, die dem zweiten Ausgang 26 zugeordnet ist.

In der zweiten, ebenfalls durch Spritzgießen aus Kunststoff hergestellten einstückigen Ventilgehäusehälfte 42 ist in der dem ersten Ausgang 24 zugeordneten ersten Differenzkraftkammer ein dritter ringförmiger Steg 58 vorgesehen, welcher den ersten Auslass 24 umgibt. Der Ringsteg 58 spannt die Membranscheibe 34 entgegen der durch die Ringstege 48 und 53 erzeugten Vorspannung in Richtung des Eingangs 20 vor. Der Durchmesser des Ringstegs 58 ist größer als der Durchmesser des ersten Ringstegs 48 und kleiner als der Durchmesser des zweiten Ringstegs 53. Die erste Differenzkraftkammer 28 wird daher durch den durch den dritten Ringsteg 58 umgrenzten Raum und die entsprechenden Anteile der Membranscheibe 34 sowie den über die Öffnung 50 verbundenen Raum in dem ersten Ringsteg 48 definiert.

Wie ferner insbesondere aus Figur 4 ersichtlich, ist das Entlastungsventil 36, über welches die erste Differenzkraftkammer 28 des ersten Ausgangs 24 zur Aussenseite geöffnet werden kann, in einem mit der zweiten Ventilgehäusehälfte 42 verbundenen Gehäuse 60 angeordnet, welches einen Anschluss 62 aufweist. Das Gehäuse 60 kann möglicherweise ebenfalls einstückig mit der zweiten Ventilgehäusehälfte 42 ausgebildet sein. Der Anschluss 62 ist entsprechend dem Anschluss 64 am Eingang 20 derart ausgebildet, dass das Vorderende einer üblichen Injektionsspritze dichtend eingeführt werden kann.

Wie ferner in den Zeichnungen gezeigt, ist bei dem erfindungsgemäßen Ausführungsbeispiel der Druckluftspeicher 14 als mit dem zweiten Ausgang 26 verbundener Gummibalg 66 ausgebildet, welcher in einem Gehäuse 68 angeordnet ist. Wie insbesondere aus Figur 4 ersichtlich, ist beim Ausführungsbeispiel der Boden 70 des Gehäuses 68 einstückig mit der zweiten Ventilgehäusehälfte 42 ausgebildet. Ein zum zweiten Ausgang 26 führender Verbindungskanal 22 ist im Boden 70 des Gehäuses mit einem geeigneten Kupplungsstück 74 versehen, welches den zweiten Ausgang 26 umgibt und welches formschlüssig mit dem Gummibalg verbunden ist. Um eine kompakte Bauweise zu erreichen, verläuft die Auslassöffnung 76 der zweiten Differenzkraftkammer 30 abgewinkelt zum Verbindungskanal 72.

Bei dem Ausführungsbeispiel ist die Membranscheibe 34 am Umfang mit einem einstückigen Ringwulst 78 versehen, welcher in einander gegenüberliegenden ringförmigen Ausnehmungen 80 und 82 entsprechenden Querschnitts in den Ventilgehäusehälften 40 und 42 aufgenommen ist, wenn die Ventilgehäusehälften 40 und 42 miteinander verbunden sind.

Das in dem Gehäuse 60 angeordnete Entlastungsventil 36 ist, da es lediglich dem Entlasten des Cuffs 8 und des Gummibalgs 66 dient, als einfaches Pilzventil 84 ausgebildet. Die den Pilzkopf bildende elastische Membran 86 deckt hierbei ein oder mehrere Verbindungskanäle 88 zwischen dem Innenraum des Gehäuses 60 und der ersten Differenzkraftkammer ab. Wenn eine Inkektionsspritze in den Anschluss 62 des Gehäuses 60 mediendicht eingeführt wird und die Spitze aufgezogen wird, so hebt der im Innenraum des Gehäuses 60 erzeugte Unterdruck die elastische Membran 86 an, so dass die Verbindungskanäle 88 freigegeben werden und der in der zweiten Differenzkraftkammer 30 hierdurch erzeugte Unterdruck die Membranscheibe 34 von dem Ringsteg 58 abhebt, so dass die in dem Cuff 8 enthaltene Luft über den Ausgang 24 und die in dem Gummibalg 66 enthaltene Luft über den Ausgang 26 entfernt werden kann.

Wird nun nach Einführen des Beatmungstubus 4 in die Trachea 2 eines Patienten über eine an den Eingang 20 bzw. dessen Anschluss 64 angeschlossene Injektionsspritze ca. 40 bis 50 ml Luft eingeführt, so wird die Membranscheibe 34 zunächst von dem ersten Ringsteg 48 und dem zweiten Ringsteg 53 abgehoben, so dass die Luft über die Öffnung 50 und die Öffnungen 56 durch die erste Differenzkraftkammer 28 und die zweite Differenzkraftkammer 30 strömen kann und sowohl der Cuff 8 als auch der Gummibalg 66 aufgeblasen werden. Nach Entfernen der Injektionsspritze kehrt die Membranscheibe 34 zurück auf ihre durch den ersten Ringsteg 48 und den zweiten Ringsteg 58 gebildeten Sitze nach Art eines Rückschlagventils, so dass die über die Verbindungsleitung 10 vom ersten Ausgang 24 zum Cuff 8 gelangte Luft und die über den zweiten Ausgang 26 in den Gummibalg 6 gelangte Luft nicht mehr nach aussen entweichen kann. In diesem Zustand befindet sich der Cuff 8 und der Gummibalg 6 hinsichtlich des Drucks in einem Gleichgewichtszustand. Sollte nun entweder durch Bewegung des Patienten oder durch einen zu hohen Druck bei der Beatmung Luft aus dem Cuff 8 verdrängt werden, so wirkt diese zunächst in der ersten Differenzkraftkammer 28 und versucht, die Membranscheibe 34 von dem dritten Ringsteg 58 abzuheben. Der Strömung der Luft wird hierbei auf Grund der zur Verfügung stehenden Fläche der Membranscheibe 34 in der ersten Differenzkraftkammer und des kleineren Strömungsquerschnitts im Ausgang 24 ein gewisser Widerstand entgegengesetzt, so dass die dichte Anlage des Cuffs 8 an der Trachea 2 gewährleistet bleibt. Nach Abheben der Membranscheibe 34 vom dritten Ringsteg 58 gelangt die Luft in die zweite Differenzkraftkammer 30 und von dort in den Gummibalg 66. Der Gummibalg 66 übernimmt hierbei die Funktion eines Druckluftspeichers 14. Lässt nun der entweder durch die Beatmung oder Bewegung des Patienten auf den Cuff 8 ausgeübte Druck nach, so ist der Druck in dem ersten Ausgang 24 kleiner als der Druck in dem zweiten Ausgang 26, so dass dieser in der zweiten Differenzkraftkammer 30 wirkende Druck wiederum die Membranscheibe 34 von dem dritten Ringsteg 58 abheben kann und die Luft zurück durch den Ausgang 24 und die Verbindungsleitung 10 in den Cuff 8 strömen kann, wobei dieser Richtung der Strömung ein wesentlich geringerer Widerstand entgegengesetzt wird als bei der Verdrängung der Luft aus dem Cuff 8, nachdem die in der zweiten Differenzkraftkammer zur Verfügung stehende Fläche der Membranscheibe 34 wesentlich größer ist.

Es ist offensichtlich, dass durch diese Konstruktion exakt das gewünschte Ansprechverhalten des Ventils erreicht wird. Es ist in diesem Zusammenhang ohne Bedeutung, ob der Druck der in Lungen gedrückten Luft kleiner oder größer ist als der Druck innerhalb des Cuffs. In beiden Fällen wird die Dichtung zwischen dem Cuff 8 und der Wandung der Trachea 2 aufrechterhalten.

Sämtliche aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

### BEZUGSZEICHENLISTE

- 1 =: Vorrichtung
- 2 =: Trachea
- 4 =: Beatmungstubus
- 6 =: distales Ende
- 8 =: Cuff
- 10 =: Verbindungsleitung
- 12 =: proximales Ende
- 14 =: Druckluftspeicher
- 16 =: Ventil
- 18 =: proximales Ende von 10
- 20 =: Eingang
- 22 =: Differenzdruckventil
- 24 =: erster Ausgang
- 26 =: zweiter Ausgang
- 28 =: erste Differenzkraftkammer
- 30 =: zweite Differenzkraftkammer
- 32 =: dritte Differenzkraftkammer
- 34 =: Membranscheibe
- 36 =: Entlastungsventil
- 38 =: Ausgang von 36
- 40 =: erste Ventilgehäusehälfte
- 42 =: zweite Ventilgehäusehälfte
- 44 =: Vorsprung
- 46 =: Oberseite von 44
- 48 =: erster Ringsteg
- 50 =: Öffnung in 34
- 52 =: Verbindungskanal
- 53 =: zweiter Ringsteg
- 54 =: Ringraum
- 56 =: Öffnung in 34
- 58 =: dritter Ringsteg
- 60 =: Gehäuse von 36
- 62 =: Anschluss von 60
- 64 =: Anschluss von 20
- 66 =: Gummibalg
- 68 =: Gehäuse von 66
- 70 =: Boden von 68
- 72 =: Verbindungskanal
- 74 =: Kupplungsstück
- 76 =: Auslassöffnung von 30
- 78 =: Ringwulst
- 80 =: Ausnehmung
- 82 =: Ausnehmung
- 84 =: Pilzventil
- 86 =: Membrane
- 88 =: Verbindungskanal

## Patentansprüche

1. Vorrichtung zur Verbindung eines Beatmungsgeräts mit dem Patienten mit einem in die Trachea (2) des Patienten einführbaren, mit dem Atemgas beaufschlagbaren Beatmungstubus (4), wie Trachealtubus bzw. Tracheostomietubus, der nahe dem distalen Ende (6) mit einem aufblasbaren Cuff (8) versehen ist, welcher eine Verbindungsleitung (10) zum proximalen Ende (12) aufweist, die mit einem elastischen Druckluftspeicher (14) verbunden ist, wobei ein mindestens drei Wege aufweisendes Ventil (16) zwischen dem proximalen Ende (18) der Verbindungsleitung (10) und dem Druckluftspeicher (14) angeordnet ist, welches einen Eingang (20) mit Anschluss (64) für eine Injektionsspritze zum Füllen des Cuffs (8) und des Druckluftspeichers (14) mit Druckluft aufweist und das Ventil (16) an dem Eingang (20) als Rückschlagventil und zur Aufrechterhaltung eines konstanten Drucks in dem Cuff (8) mittels Ausgleich zwischen Cuff (8) und dem Druckluftspeicher (14) als Steuerventil wirksam ist, **dadurch gekennzeichnet, dass** das Ventil (16) als Dreiwege-Rückschlag- und als DifferenzdruckVentil (22) ausgebildet ist, wobei das Ventil (22) mindestens zwei Ausgänge (24, 26) mit je einer zugeordneten Differenzkraftkammer (28, 30) aufweist und der Eingang (20) mit einer dritten Differenzkraftkammer (32) versehen ist, dass die Differenzkraftkammern (28, 30, 32) durch eine elastische Membranscheibe (34) dichtend voneinander getrennt sind, und dass der erste Ausgang (24) an die Verbindungsleitung (10) und der zweite Ausgang (26) an den Druckluftspeicher (14) angeschlossen ist.

2. Vorrichtung nach Anspruch 1, **dadurch gekennzeichnet, dass** die erste Differenzkraftkammer (28) des ersten Ausgangs (24) mit einem Entlastungsventil (36) verbunden ist.

3. Vorrichtung nach Anspruch 2, **dadurch gekennzeichnet, dass** das Entlastungsventil (36) zum Entspannen des Cuffs (8) durch an dessen Ausgang (38) erzeugten Unterdruck betätigbar ist.

4. Vorrichtung nach einem der vorstehenden Ansprüche, **dadurch gekennzeichnet, dass** das Differenzdruckventil (22) aus zwei dichtend miteinander verbindbaren Ventilgehäusehälften (40, 42) gebildet ist, wobei die erste Ventilgehäusehälfte (40) den Eingang (20) und die zweite Ventilgehäusehälfte (42) die beiden Ausgänge (24, 26) und das Entlastungsventil (36) enthält.

5. Vorrichtung nach Anspruch 4, **dadurch gekennzeichnet, dass** die Membranscheibe (34) zwischen der ersten und der zweiten Ventilgehäusehälfte (40, 42) eingespannt ist.

6. Vorrichtung nach Anspruch 4 oder 5, **dadurch gekennzeichnet, dass** die erste Ventilgehäusehälfte (40) innerhalb der dritten Differenzkraftkammer (32) einen Vorsprung (44) aufweist, welcher auf seinem Oberende (46) einen ersten ringförmigen Steg (48) trägt, der die Membranscheibe (34) in Richtung des ersten Ausgangs (24) vorspannt und eine in der Membranscheibe (34) vorgesehene Öffnung (50) umgibt, wobei radial ausserhalb des Vorsprungs (44) Verbindungskanäle (52) zwischen dem Einlass (20) und der dritten Differenzkraftkammer (32) vorgesehen sind, und dass die dritte Differenzkraftkammer (32) durch einen zweiten ringförmigen Steg (53) mit größerem Durchmesser als der erste Ringsteg (48) am Umfang begrenzt ist, welcher die Membranscheibe (34) in Richtung des ersten Ausgangs (24) vorspannt.

7. Vorrichtung nach Anspruch 6, **dadurch gekennzeichnet, dass** der erste Ausgang (24) parallel oder koaxial zum Eingang (20) angeordnet ist.

8. Vorrichtung nach Anspruch 6 oder 7, **dadurch gekennzeichnet, dass** radial ausserhalb des zweiten Ringstegs (53) mit größerem Durchmesser ein Ringraum (54) vorgesehen ist, welcher über Öffnungen (56) in der Membranscheibe (34) mit der dem zweiten Ausgang (26) zugeordneten zweiten Differenzkraftkammer (30) verbunden ist.

9. Vorrichtung nach einem der Ansprüche 4 bis 8, **dadurch gekennzeichnet, dass** in der dem ersten Ausgang zugeordneten ersten Differenzkraftkammer (28) ein den ersten Auslass (24) umgebender dritter ringförmiger Steg (58) vorgesehen ist, welcher die Membranscheibe (34) in Richtung des Eingangs (20) vorspannt und dessen Durchmesser größer als der Durchmesser des ersten Ringstegs (48) und kleiner als der Durchmesser des zweiten Ringstegs (53) ist.

10. Vorrichtung nach einem der Ansprüche 2 bis 9, **dadurch gekennzeichnet, dass** das Entlastungsventil (36) in einem einen Anschluss (62) aufweisenden, mit der zweiten Ventilgehäusehälfte (42) verbundenen Gehäuse (60) angeordnet ist.

11. Vorrichtung nach Anspruch 10, **dadurch gekennzeichnet, dass** der Anschluss (62) des Gehäuses (60) und der Anschluss (64) des Eingangs (20) zum Einführen des Vorderendes einer Injektionsspritze ausgebildet sind.

12. Vorrichtung nach einem der vorstehenden Ansprüche, **dadurch gekennzeichnet, dass** der Druckluftspeicher (14) als mit dem zweiten Ausgang (26) verbundener Gummibalg (66) ausgebildet ist.

13. Vorrichtung nach Anspruch 12, **dadurch gekennzeichnet, dass** der Gummibalg (66) in einem Gehäuse (68) aufgenommen ist.

14. Vorrichtung nach Anspruch 13, **dadurch gekennzeichnet, dass** zumindest der Boden (70) des Gehäuses (68) einstückig mit der zweiten Ventilgehäusehälfte (42) ausgebildet und über ein einen Verbindungskanal (72) zum zweiten Ausgang (26) umgebendes Kupplungsstück (74) mit dem Gummibalg (66) verbunden ist.

15. Vorrichtung nach Anspruch 14, **dadurch gekennzeichnet, dass** die Auslassöffnung (76) der zweiten Differenzkraftkammer (30) abgewinkelt zum Verbindungskanal (72) verläuft.

16. Vorrichtung nach einem der vorstehenden Ansprüche, **dadurch gekennzeichnet, dass** die Membranscheibe (34) am Umfang einen Ringwulst (78) aufweist, welcher in einander gegenüberliegenden ringförmigen Ausnehmungen (80, 82) der Ventilgehäusehälften (40, 42) aufgenommen ist.

17. Vorrichtung nach einem der Ansprüche 2 bis 16, **dadurch gekennzeichnet, dass** das Entlastungsventil (36) als Pilzventil (84) ausgebildet ist, dessen elastische Membran (86) einen oder mehrere Verbindungskanäle (88) zur ersten Differenzkraftkammer (28) abdeckt.

## Claims

1. Apparatus for connecting a respiratory device with a patient having a breathing tube (4), e.g. an endotracheal tube or tracheostomy tube, insertable into the trachea (2) of a patient and being connectable to a source of breathing gas and having an inflatable cuff (8) close to the distal end (6) thereof which is having a connection line (10) to the proximal end (12) thereof connected to an elastic pressurized air reservoir (14), wherein a valve (16) having at least three ways is positioned between the proximal end (18) of the connection line (10) and the pressurized air reservoir (14) which is having one entry (20) with a connection (64) for a syringe for filling the cuff (8) and the pressurized air reservoir (14) with pressurized air, the valve (16) at the entry (20) being effective as a check valve and being effective as a control valve for maintaining a constant pressure within the cuff (8) by means of compensation between the cuff (8) and the pressurized air reservoir (14), **characterized in that** the valve (16) is performed as a three-way check valve and as a differential pressure valve (22), wherein the valve (22) at least is having two exits (24, 26) with each an associated differential force chamber (28, 30) with the entry (20) being provided with a third differential force chamber (32), that the differential force chambers (28, 30, 32) are sealingly separated from another by a flexible membrane disk (34) and, **in that** the first exit (24) is connected to the connection line (10) and the second exit (26) is connected to the pressurized air reservoir (14).

2. Apparatus according to claim 1, **characterized in that** the first differential force chamber (28) of the first exit (24) is connected to a relief valve (36).

3. Apparatus according to claim 2, **characterized in that** the relief valve (36) can be actuated by a vacuum created at the exit (38) thereof for the decompression of the cuff (8).

4. Apparatus according to any of the preceding claims, **characterized in that** the differential pressure valve (22) is consisting of two valve housing halves (40, 42) sealingly connectable with each other, wherein the first valve housing half (40) is containing the entry (20) and the second valve housing half (42) is containing the two exits (24, 26) and the relief valve (36).

5. Apparatus according to claim 4, **characterized in that** the membrane disk (34) is clamped between the first and second valve housing halves (40, 42).

6. Apparatus according to claim 4 or 5, **characterized in that** the first valve housing half (40) within the third differential force chamber (32) is having a projection (44) which on its top end (46) is carrying a first annular web (48) which is pretensioning the membrane disk (34) in the direction of the first exit (24) and which is surrounding an opening (50) provided in the membrane disk (34), wherein radially beyond the projection (44) there are provided connection channels (52) between the entry (20) and the third differential force chamber (32) and, **in that** the third differential force chamber (32) is limited at the circumference thereof by a second annular web (53) having a larger diameter than the first annular web (48) which is pretensioning the membrane disk (34) in the direction of the first exit (24).

7. Apparatus according to claim 6, **characterized in that** the first exit (24) is positioned in parallel or coaxially to the entry (20).

8. Apparatus according to claim 6 or 7, **characterized in that** radially beyond the second annular web (53) having the larger diameter there is provided an annular space (54) which by means of openings (56) in the membrane disk (34) is connected with the second differential force chamber (30) associated with the second exit (26).

9. Apparatus according to any of the claims 4 to 8, **characterized in that** in the first differential force chamber (28) associated with the first exit there is provided a third annular web (58) surrounding the first exit (24) which is pretensioning the membrane , disk (34) in the direction of the entry (20) and the diameter of which being larger than the diameter of the first annular web (48) and smaller than the diameter of the second annular web (53).

10. Apparatus according to any of the claims 2 to 9, **characterized in that** the relief valve (36) is positioned in a housing (60) having a connector (62) and being connected to the second valve housing half (42).

11. Apparatus according to claim 10, **characterized in that** the connector (62) of the housing (60) and the connector (64) of the entry (20) is suitable for the insertion of the front end of a syringe.

12. Apparatus according to any of the preceding claims, **characterized in that** the pressurized air reservoir (14) is performed as a rubber bellows (66) connected with the second exit (26).

13. Apparatus according to claim 12, **characterized in that** the rubber bellows (66) is contained within a housing (68).

14. Apparatus according to claim 13, **characterized in that** at least the bottom (70) of the housing (68) is monolithic with the second valve housing half (42) and is connected with the rubber bellows (66) by means of a coupling member (74) containing a connection channel (72) leading to the second exit (26).

15. Apparatus according to claim 14, **characterized in that** the exit opening (76) of the second differential force chamber (30) is extending in an angle with respect to the connection channel (72).

16. Apparatus according to any of the preceding claims, **characterized in that** the membrane disk (34) is having an annular protrusion (78) at the circumference thereof which is received within opposed annular recesses (80, 82) of the valve housing halves (40, 42).

17. Apparatus according to any of the claims 2 to 16, **characterized in that** the relief valve (36) is performed as an umbrella valve (84), the elastic membrane (86) thereof covering one or more connection channels (88) leading to the first differential force chamber (28).

## Revendications

1. Dispositif pour connecter un appareil respiratoire à un patient, comprenant un tube respiratoire (4) pouvant être introduit dans la trachée (2) du patient et pouvant être sollicité avec le gaz respiratoire, tel qu'un tube trachéal ou un tube de trachéotomie, qui est pourvu, à proximité de l'extrémité distale (6), d'un ballonnet gonflable (8), qui présente une conduite de connexion (10) à l'extrémité proximale (12), laquelle est connectée à un accumulateur d'air sous pression élastique (14), une soupape (16) présentant au moins trois voies étant disposée entre l'extrémité proximale (18) de la conduite de connexion (10) et l'accumulateur d'air sous pression (14), laquelle présente une entrée (20) avec un raccord (64) pour une seringue d'injection pour remplir le ballonnet (8) et l'accumulateur d'air sous pression (14) avec de l'air sous pression, et la soupape (16) agissant à l'entrée (20) en tant que clapet anti-retour et, pour maintenir une pression constante dans le ballonnet (8), au moyen d'un équilibrage entre le ballonnet (8) et l'accumulateur d'air sous pression (14), en tant que soupape de commande, **caractérisé en ce que** la soupape (16) est réalisée sous forme de clapet anti-retour à trois voies et en tant que soupape à pression différentielle (22), la soupape (22) présentant au moins deux sorties (24, 26) ayant chacune une chambre de force différentielle associée (28, 30) et l'entrée (20) étant pourvue d'une troisième chambre de force différentielle (32), **en ce que** les chambres de force différentielle (28, 30, 32) sont séparées hermétiquement les unes des autres par un disque de membrane élastique (34), et **en ce que** la première sortie (24) est raccordée à la conduite de connexion (10) et la deuxième sortie (26) est raccordée à l'accumulateur d'air sous pression (14).

2. Dispositif selon la revendication 1, **caractérisé en ce que** la première chambre de force différentielle (28) de la première sortie (24) est connectée à une soupape de détente (36).

3. Dispositif selon la revendication 2, **caractérisé en ce que** la soupape de détente (36) peut être actionnée pour détendre le ballonnet (8) par la dépression produite au niveau de sa sortie (38).

4. Dispositif selon l'une quelconque des revendications précédentes, **caractérisé en ce que** la soupape à pression différentielle (22) est formée de deux moitiés de boîtier de soupape (40, 42) pouvant être connectées hermétiquement l'une à l'autre, la première moitié de boîtier de soupape (40) contenant l'entrée (20) et la deuxième moitié de boîtier de soupape (42) contenant les deux sorties (24, 26) et la soupape de détente (36).

5. Dispositif selon la revendication 4, **caractérisé en ce que** le disque de membrane (34) est serré entre la première et la deuxième moitié de boîtier de soupape (40, 42).

6. Dispositif selon la revendication 4 ou 5, **caractérisé en ce que** la première moitié de boîtier de soupape (40) présente à l'intérieur de la troisième chambre de force différentielle (32) une saillie (44) qui porte à son extrémité supérieure (46) une première nervure annulaire (48), qui précontraint le disque de membrane (34) dans la direction de la première sortie (24) et entoure une ouverture (50) prévue dans le disque de membrane (34), des canaux de connexion (52) entre l'entrée (20) et la troisième chambre de force différentielle (32) étant prévus radialement à l'extérieur de la saillie (44), et **en ce que** la troisième chambre de force différentielle (32) est limitée à sa périphérie par une deuxième nervure annulaire (53) de plus grand diamètre que la première nervure annulaire (48), laquelle précontraint le disque de membrane (34) dans la direction de la première sortie (24).

7. Dispositif selon la revendication 6, **caractérisé en ce que** la première sortie (24) est disposée parallèlement ou coaxialement à l'entrée (20).

8. Dispositif selon la revendication 6 ou 7, **caractérisé en ce qu'**un espace annulaire (54) est prévu radialement en dehors de la deuxième nervure annulaire (53) de plus grand diamètre, lequel est connecté par le biais d'ouvertures (56) dans le disque de membrane (34) à la deuxième chambre de force différentielle (30) associée à la deuxième sortie (26).

9. Dispositif selon l'une quelconque des revendications 4 à 8, **caractérisé en ce que** dans la première chambre de force différentielle (28) associée à la première sortie est prévue une troisième nervure annulaire (58) entourant la première sortie (24), laquelle précontraint le disque de membrane (34) dans la direction de l'entrée (20) et dont le diamètre est supérieur au diamètre de la première nervure annulaire (48) et inférieur au diamètre de la deuxième nervure annulaire (53).

10. Dispositif selon l'une quelconque des revendications 2 à 9, **caractérisé en ce que** la soupape de détente (36) est disposée dans un boîtier (60) présentant un raccord (62), connecté à la deuxième moitié de boîtier de soupape (42).

11. Dispositif selon la revendication 10, **caractérisé en ce que** le raccord (62) du boîtier (60) et le raccord (64) de l'entrée (20) sont réalisés de manière à introduire l'extrémité avant d'une seringue d'injection.

12. Dispositif selon l'une quelconque des revendications précédentes, **caractérisé en ce que** l'accumulateur d'air sous pression (14) est réalisé sous forme de soufflet en caoutchouc (66) connecté à la deuxième sortie (26).

13. Dispositif selon la revendication 12, **caractérisé en ce que** le soufflet en caoutchouc (66) est reçu dans un boîtier (68).

14. Dispositif selon la revendication 13, **caractérisé en ce qu'**au moins le fond (70) du boîtier (68) est réalisé d'une seule pièce avec la deuxième moitié de boîtier de soupape (42) et est connecté au soufflet en caoutchouc (66) par le biais d'une pièce d'accouplement (74) entourant un canal de connexion (72) vers la deuxième sortie (26).

15. Dispositif selon la revendication 14, **caractérisé en ce que** l'ouverture de sortie (76) de la deuxième chambre de force différentielle (30) s'étend de manière coudée par rapport au canal de connexion (72).

16. Dispositif selon l'une quelconque des revendications précédentes, **caractérisé en ce que** le disque de membrane (34) présente sur sa périphérie un bourrelet annulaire (78) qui est reçu dans des évidements de forme annulaire (80, 82) opposés les uns aux autres des moitiés de boîtier de soupape (40, 42).

17. Dispositif selon l'une quelconque des revendications 2 à 16, **caractérisé en ce que** la soupape de détente (36) est réalisée sous forme de soupape à champignon (84), dont la membrane élastique (86) recouvre un ou plusieurs canaux de connexion (88) vers la première chambre de force différentielle (28).
